# EUROPÄISCHE PATENTSCHRIFT

(11) **EP 0 306 723 B1**
(45) Veröffentlichungstag und Bekanntmachung des Hinweises auf die Patenterteilung: **10.03.1993**
(21) Anmeldenummer: 88113050.4
(22) Anmeldetag: 11.08.1988
(51) Int. Cl.: A61B 1/00

(54) **Steuervorrichtung zur Ablenkung des distalen Endes eines Endoskopes**
Control device for deflecting the distal end of an endoscope
Dispositif de commande pour courber l'extrémité distale d'un endoscope

(30) Priorität: 01.09.1987 DE 3729131
(43) Veröffentlichungstag der Anmeldung: 15.03.1989
(73) Patentinhaber: Richard Wolf GmbH, 75438 Knittlingen (DE)
(72) Erfinder: Heimberger, Rudolf, D-7519 Oberderdingen (DE)
(74) Vertreter: Wilcken, Thomas, Dipl.-Ing.

(56) Entgegenhaltungen:
- DE-A- 2 557 545
- US-A- 3 557 780
- US-A- 4 461 282
- US-A- 4 617 914

## Beschreibung

Die Erfindung geht aus von einer mechanischen Steuervorrichtung zum Ablenken des distalen Endes eines flexiblen Endoskopes in mindestens einer Ebene durch Betätigen von zum distalen Ende verlaufenden Steuerseilen mittels einer Handhabe, wobei die Steuervorrichtung von einem Steuergehäuse am proximalen Ende des Endoskopes aufgenommen wird und einen Verstell- und Bremsmechanismus zum Verstellen und Fixieren des genannten distalen Endes aufweist.

Aus der DE-A-25 57 545 ist ein flexibles Endoskop mit einer kombinierten Brems- und Steuervorrichtung der vorgenannten Art bekannt, das zum Zwecke der beiderseitigen Ablenkung des distalen Endoskopendes am proximalseitig angeordneten Steuergehäuse ein Handrad mit einer Druckknopf-Entriegelung aufweist. Soll nach erfolgter Einstellung der Abwinkelung des distalen Instrumentenendes dieses in der eingestellten Position verbleiben, so kann durch einen gesondert zu betätigenden Bremshebel eine weitere Verstellung oder eine Rückkehr in die gestreckte Ausgangslage wirksam verhindert werden. Erfordert beispielsweise die Untersuchung einer Körperhöhle eine Feineinstellung des distalen Instrumentenendes, so kann durch Betätigen des Druckknopfes an dem Handrad die Arretierung des distalen Instrumentenendes durch eine Bremsscheibe aufgehoben und das Handrad verstellt werden. Eine Feinverstellung ist so lange möglich, wie der Druckknopf betätigt wird.

Wenn zwar einerseits durch die Kombination des Handrades mit dem Druckknopf eine Vereinfachung erzielt wird, so sind aber andererseits zum sicheren gleichzeitigen Betätigen des Bremshebels, des Druckknopfes und des Handrades sowie für das Festhalten des Steuergehäuses zwei Hände erforderlich, um die gewünschte Manipulation ausführen zu können. Zumindest ist aber eine Einhandbetätigung sehr umständlich und erfordert sehr viel Geschick.

Zur Vermeidung eines derartigen Nachteiles wird in der DE-B 26 03 370 eine Verstelleinrichtung zum allseitigen Abwinkeln des distalen Endoskopendes vorgeschlagen, die sowohl um ihre Drehachse verdreht als auch verschwenkt werden kann. Eine Bremse weist der hier beschriebene Verstelleinrichtung jedoch nicht auf, womit eine Feststellung des steuerbaren distalen Endoskopendes in der gewünschten Position nicht möglich ist.

Ein weiterer Nachteil des bekannten Standes der Technik besteht hinsichtlich der Ablenkung des distalen Endoskopendes mittels der am proximalseitigen Steuergehäuse angeordneten Verstelleinrichtung darin, daß die Einhandbedienung nur in einem begrenzten Bereich möglich ist. Ist es jedoch aus bestimmten Gründen erforderlich, eine diesen Bereich überschreitende Verstellung vorzunehmen, so läßt es sich nicht vermeiden, zusätzlich auch die zweite Hand zur Betätigung der Verstelleinrichtung zu benutzen.

Der Erfindung liegt die Aufgabe zugrunde, eine Steuereinrichtung zum Ablenken des distalen Endoskopendes in mindestens einer Ebene so auszubilden, daß sowohl das Ablenken über den gesamten Bereich als auch das Festlegen des abgewinkelten Endoskopendes in jeder beliebigen Lage einfach, schnell und sicher mit einer Hand vorgenommen werden kann.

Diese Aufgabe wird ausgehend von der eingangs erwähnten Steuervorrichtung erfindungsgemäß so gelöst, daß die Steuervorrichtung einen einhändig bedienbaren, als kombinierten Brems- und Steuerhebel dienenden Schwenkhebel als Handhabe aufweist, der beim Verdrehen um eine erste Achse über einen im Steuergehäuse drehbar gelagerten Einsatz auf die Steuerseile einwirkt und der um eine zweite Achse zwischen einer ersten Stellung, in der er die Verstellbarkeit des distalen Endes des Endoskopes ermöglicht, und einer zweiten Stellung, in der er eine Fixierung desselben bewirkt, schwenkbar ist.

Der Schwenkhebel ragt zweckmäßigerweise mit seinem Bedienteil frei über das Steuergehäuse hinaus und ist in seiner ersten Stellung durch ein Rastelement lösbar verriegelbar. Unter besonderer Beachtung ergonomischer Gesichtspunkte bei der Gestaltung des Steuergehäuses und des Schwenkhebels sind damit einerseits eine gute Handhabung und andererseits sowohl die Steuerung als auch das Festlegen des distalseitig ablenkbaren Endoskopendes einfach, schnell und sicher mit nur einer Hand möglich, was auch dadurch noch unterstützt wird, daß die Fixierung des Schwenkhebels in der zweiten Stellung durch eine Druckfeder erfolgt, die den Schwenkhebel über ein Hebelsystem in dieser Stellung festhält und den vorerwähnten Einsatz in Reibschluß mit einer gehäusefesten Fläche hält.

Die Erfindung ist nachfolgend anhand eines in der Zeichnung dargestellten Ausführungsbeispieles näher erläutert. es zeigen:
- Figur 1: einen Teil des proximalseitigen Steuergehäuses in perspektivischer Darstellung,

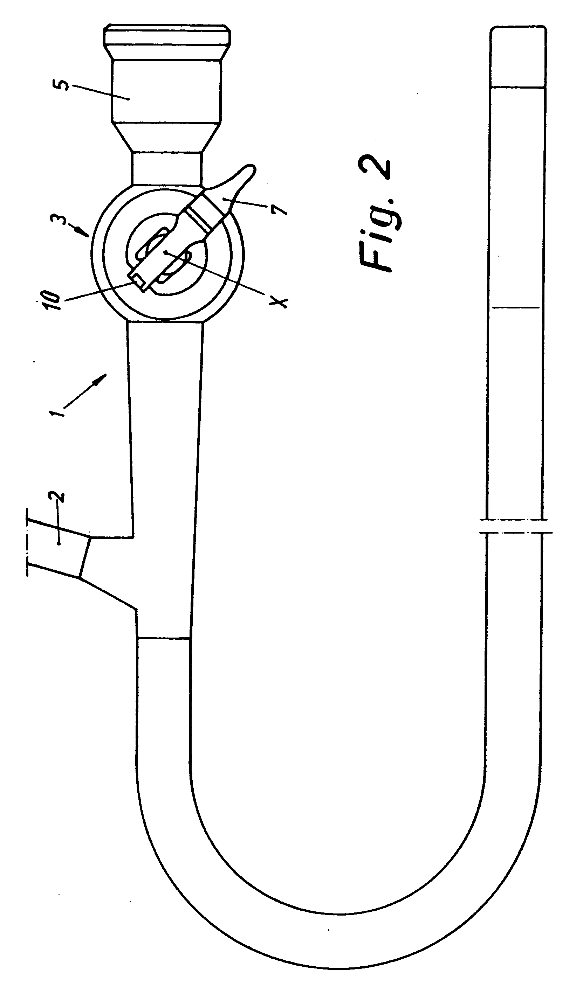

- Figur 2: eine Ansicht des proximalseitigen Steuergehäuses mit angeschlossenem Endoskop,
- Figur 3: einen Längsschnitt durch die erfindungsgemäße Steuervorrichtung in stark vergrößertem Maßstab.

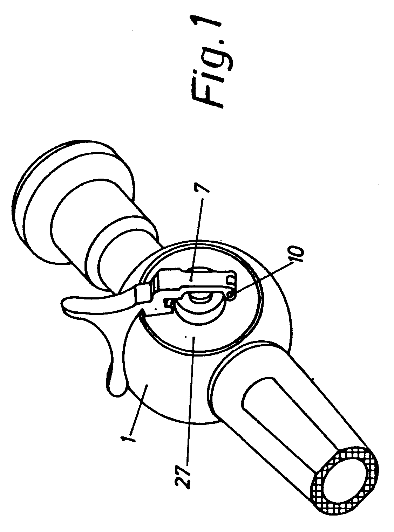

In Figur 1 ist das proximalseitige Ende eines flexiblen Endoskopes dargestellt, das ein Steuergehäuse 1 aufweist, in bzw. an dem das eine Ende eines Lichtleitkabels 2, eine Steuervorrichtung 3 mit dem als Brems- und Steuerhebel dienenden, um eine Achse x verdrehbaren Schwenkhebel 7 sowie ein Okular mit dem Okulartrichter 5 angeordnet sind.

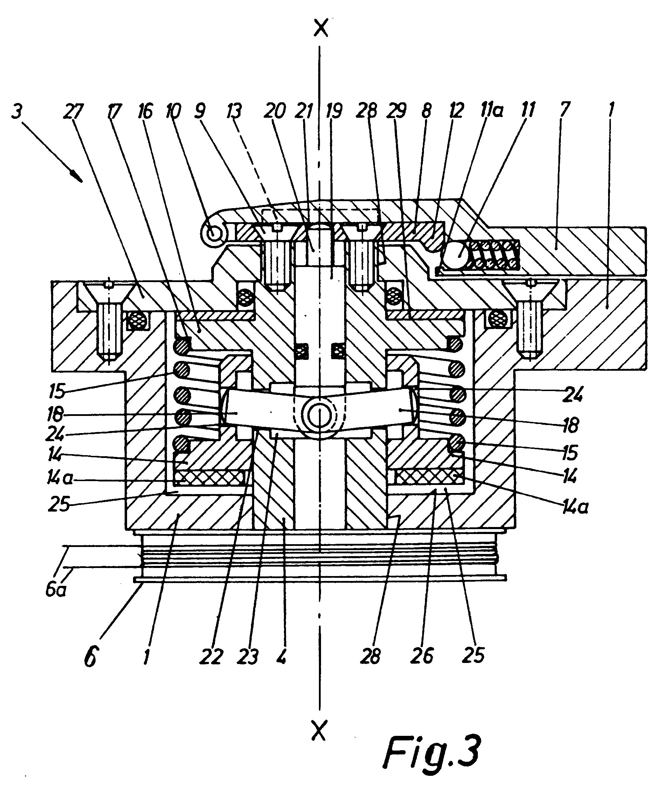

Die Steuervorrichtung 3 weist nach Figur 3 einen Hohlzylinder 4 auf, dessen oberes Ende eine Nut zur Aufnahme einer Verschlußplatte 8 und zur Führung des Schwenkhebels 7 hat und dessen unteres Ende mit einer Rolle 6 verbunden ist, die zur Aufnahme der bis zum distalen Endoskopende verlaufenden Steuerseile 6a dient. Die in die Nut des Hohlzylinders 4 eingreifende Verschlußplatte 8 ist durch zwei Schrauben 9 auf dem Hohlzylinder 4 festgelegt.

Über ein eine zweite Achse bildendes Gelenk 10 ist der Schwenkhebel 7 schwenkbeweglich mit der Verschlußplatte 8 verbunden, wobei eine abgefederte Rastkugel 11 eine dem Gelenk diametral gegenüberliegende Nase 12 hintergreift, um den Schwenkhebel 7 ungebremst in der ersten Stellung gegen ein Ausschwenken zu fixieren. Durch einen unterhalb der Kugel 11 befindlichen Anschlag 11a wird der Schwenkwinkel des Schwenkhebels 7 nach unten begrenzt. Zur Vermeidung von auf das Gelenk 10 bei der Verdrehung des Schwenkhebels 7 um die Achse x wirkenden Scherkräften greift dieser, wie bereits erwähnt wurde, in die Stirnnut des Hohlzylinders 4 ein und wird durch die beiden die Drehbewegung um die Achse x übertragenden, die Verschlußplatte 8 durchgreifenden und durch die Nut gebildeten Wandungsteile 13 des Hohlzylinders 4 geführt.

Zur Abstützung einer Druckfeder 15 weist der Hohlzylinder 4 eine Schulter 16 mit einer einseitigen Umfangsausnehmung 17 auf, in welche die Feder 15 mit ihrem einen Ende eingreift. Unterhalb der Schulter 16 ist ein eine Bremsscheibe 14a tragender Bremskörper 14 drehbar um die Achse x und axial verschiebbar angeordnet und über zwei Hebelarme 18 und eine mit diesen gelenkig verbundene, in der zentralen Bohrung des Hohlzylinders 4 geführte Schubstange 19 betätigbar, die in dieser Bohrung längsverschiebbar angeordnet ist und durch die Druckfeder 15 und die beiden Hebelarme 18 mit dem im Durchmesser verringerten Ende 20 durch eine in der Verschlußplatte 8 befindliche Bohrung 21 hindurch gegen den Schwenkhebel 7 vorgespannt wird. Die Hebelarme 18 greifen durch zwei diametral zueinander angeordnete Ausnehmungen 22, welche in einer Nut 23 der Bohrung des Hohlzylinders 4 münden und Umlenkpunkte für die einerseits mit der Schubstange 19 gelenkig verbundenen und andererseits jeweils in eine Bohrung 24 des Bremskörpers 14 eingreifenden Hebelarme 18 bilden.

Der Hohlzylinder 4, der darauf längsverschiebbare Bremskörper 14 mit der Druckfeder 15, die im Hohlzylinder angeordnete und axial verschiebbare Schubstange 19 sowie die mit dem Hohlzylinder lösbar verbundene Verschlußplatte 8 bilden gemeinsam mit dem Schwenkhebel 7 im wesentlichen die Brems- und Steuervorrichtung 3, welche in Bohrungen 28 des diese Funktionseinheit aufnehmenden, mit einer Abdeckung 27 versehenen Steuergehäuses 1 drehbeweglich gelagert ist. Um eine bewegungshemmende Reibung weitgehend zu vermeiden, ist zwischen der Schulter 16 und der Abdeckung 27 ein Ring 29 angeordnet, der beispielsweise aus Kunststoff mit niedrigem Reibungskoeffizienten gefertigt sein kann. Um zu vermeiden, daß bei der Desinfektion des Endoskopes flüssiges Desinfektionsmittel in das Steuergehäuse 1 und in die Steuervorrichtung 3 gelangen kann, sind entsprechend Figur 3 an allen Trennstellen Dichtringe angeordnet.

Die Handhabung der erfindungsgemäßen Steuervorrichtung 3 ist sehr einfach und kann der gestellten Aufgabe entsprechend bequem mit nur einer Hand erfolgen. So kann das distale Endoskopende bei dem in Figur 3 dargestellten Betriebszustand der Steuervorrichtung durch Verdrehen des Schwenkhebels 7 um die Mittelachse x in einer Ebene beliebig abgewinkelt werden, da sich zwischen der Bremsscheibe 14a und der Bodenfläche 26 des Steuergehäuses 1 ein geringer Spalt 25 befindet. Die Rolle 6 kann also zur Verstellung der Steuerseite 6a ungebremst verdreht werden.

Wird der Schwenkhebel 7 mit geringem Kraftaufwand so weit vom Steuergehäuse 1 auswärts bzw. gemäß Figur 3 nach oben in seine zweite Stellung um die Achse 10 verschwenkt, bis der Anschlag 11a an der Nase 12 anliegt, so wird der zwischen dem Schwenkhebel 7 und der Verschlußplatte 8 bestehende Kraftschluß aufgehoben, da sich die Kugel 11 nicht mehr in unmittelbarem Eingriff mit der Nase 12 befindet. Mit der auswärts gerichteten Verschwenkung des Schwenkhebels 7 kann sich auch die durch die Feder 15 vorgespannte Schubstange 19 selbsttätig aus dem Hohlzylinder 4 herausschieben, wodurch die beiden in den Ausnehmungen 24 schwenkbeweglich gelagerten Hebelarme 18 den Bremskörper 14 in Richtung auf die Bodenfläche 26 hin verschieben, bis die Bremsscheibe 14a auf dieser Bodenfläche kraftschlüssig zu liegen kommt.

Durch die jetzt auf den Bremskörper und die Bremsscheibe wirkende Druckkraft der Feder 15 wird das distal abgewinkelte Endoskopende in dem Maße festgelegt und fixiert, daß zwar seine selbsttätige Rückstellung in die ursprüngliche Ausgangslage vermieden, eine gegebenenfalls aber doch erforderliche geringfügige und willkürliche Verstellung jedoch möglich bleibt. Sollte die Bremswirkung erhöht werden müssen, so kann dies beispielsweise auch einfach durch Anbringen einer zusätzlichen Bremsscheibe auf der Bodenfläche 26 erfolgen. Andererseits ist es auch möglich, eine stärkere Druckfeder zu verwenden oder eine in ihrer Federkraft einstellbare Druckfeder vorzusehen.

Im übrigen kann die Fixierung des distalen Endoskopendes natürlich einfach aufgehoben werden, indem der Schwenkhebel 7 wieder von Hand in die erste und der Darstellung entsprechend gehäusenahe Stellung gebracht wird, wodurch die jeweiligen Bauteile wieder ihre in Figur 3 dargestellte Position einnehmen werden.

## Patentansprüche

1. Mechanische Steuervorrichtung zum Ablenken des distalen Endes eines flexiblen Endoskopes in mindestens einer Ebene durch Betätigen von zum distalen Ende verlaufenden Steuerseilen mittels einer Handhabe, wobei die Steuervorrichtung von einem Steuergehäuse am proximalen Ende des Endoskopes aufgenommen wird und einen Verstell- und Bremsmechanismus zum Verstellen und Fixieren des genannten distalen Endes aufweist, dadurch gekennzeichnet, daß die Steuervorrichtung einen einhändig bedienbaren, als kombinierten Brems- und Steuerhebel dienenden Schwenkhebel (7) als Handhabe aufweist, der beim Verdrehen um eine erste Achse (x) über einen im Steuergehäuse (1) drehbar gelagerten Einsatz (4, 6) auf die Steuerseile (6a) einwirkt und der um eine zweite Achse (10) zwischen einer ersten Stellung, in der er die Verstellbarkeit des distalen Endes des Endoskopes ermöglicht, und einer zweiten Stellung, in der er eine Fixierung desselben bewirkt, schwenkbar ist.

2. Steuervorrichtung nach Anspruch 1, dadurch gekennzeichnet, daß der Schwenkhebel (7) mit seinem Bedienteil frei über das Steuergehäuse (1) hinausragt und in seiner ersten Stellung durch ein Rastelement (11) lösbar verriegelt ist.

3. Steuergehäuse nach einem der Ansprüche 1 oder 2, dadurch gekennzeichnet, daß die Fixierung des Schwenkhebels (7) in der zweiten Stellung durch eine Druckfeder (15) erfolgt, die den Schwenkhebel über ein Hebelsystem (19, 20) in dieser Stellung fixiert und den genannten Einsatz (4, 6) in Reibschluß mit einer gehäusefesten Fläche (26) hält.

## Claims

1. A mechanical control device for deflecting the distal end of a flexible endoscope in at least one plane by actuating control cables, running to the distal end, by means of a handle, in which the control device is held by a control housing at the proximal end of the endoscope and has an adjustment- and braking mechanism for adjusting and fixing the said distal end, characterised in that the control device has as a handle a rocking lever (7) which is able to be operated with one hand, serving as combined braking- and control lever, which on turning about a first axis (x) acts on the control cable (6a) via an insert (4,6) which is rotatably mounted in the control housing (1), and which is orientable about a second axis (10) between a first position, in which it makes possible the adjustability of the distal end of the endoscope, and a second position, in which it brings about a fixing thereof.

2. A control device according to Claim 1, characterised in that the rocking lever (7) projects with its operating part freely over the control housing (1) and in its first position is detachably locked by a detent element (11).

3. A control housing according to one of Claims 1 or 2, characterised in that the fixing of the rocking lever (7) takes place in the second position by a compression spring (15), which fixes the rocking lever in this position via a lever system (19,20) and holds the said insert (4, 6) in friction-locking arrangement with a surface (26) which is fixed to the housing.

## Revendications

1. Dispositif de commande mécanique pour faire dévier l'extrémité distale d'un endoscope flexible dans au moins un plan par actionnement de câbles de commande qui s'étendent jusqu'à l'extrémité distale, à l'aide d'une manette, le dispositif de commande étant logé dans un boîtier de commande sur l'extrémité proximale de l'endoscope et comportant un mécanisme de réglage et de freinage servant à régler et fixer ladite extrémité distale, caractérisé en ce que le dispositif de commande comporte, comme manette, un levier pivotant (7) qui peut être actionné d'une main, est utilisé en tant que levier combiné de freinage et de commande et, lors de la rotation autour d'un premier axe (x), agit sur le câble de commande (6a) par l'intermédiaire d'un insert (4,6) monté rotatif dans le boîtier de commande (1) et peut pivoter autour d'un second axe (10) entre une première position, dans laquelle il permet le réglage de l'extrémité distale de l'endoscope, et une seconde position dans laquelle il bloque cette extrémité.

2. Dispositif de commande selon la revendication 1, caractérisé en ce que le levier pivotant (7) fait saillie librement, par sa partie de commande, hors du boîtier de commande (1) et est verrouillé de façon amovible, dans sa première position, par un élément d'encliquetage (11).

3. Boîtier de commande selon l'une des revendications 1 ou 2, caractérisé en ce que le blocage du levier pivotant (7) dans la seconde position est réalisé au moyen d'un ressort de pression (15), qui bloque dans cette position le levier pivotant par l'intermédiaire d'un système à leviers (19,20) et maintient ledit insert (4,6) appliqué selon une liaison à frottement contre une surface (26) solidaire du boîtier.
